(19) **Europäisches Patentamt**

**European Patent Office**

**Office européen des brevets**

(11) **EP 1 091 979 B1**

(12) **EUROPEAN PATENT SPECIFICATION**

(45) Date of publication and mention
of the grant of the patent:
**20.09.2006 Bulletin 2006/38**

(21) Application number: **99931016.2**

(22) Date of filing: **30.06.1999**

(51) Int Cl.:
*C07K 14/62* (2006.01)     *C30B 29/58* (2006.01)

(86) International application number:
**PCT/DK1999/000371**

(87) International publication number:
**WO 2000/001727 (13.01.2000 Gazette 2000/02)**

(54) **SEEDING CRYSTALS FOR THE PREPARATION OF PEPTIDES OR PROTEINS**

IMPFKRISTALLE ZUR HERSTELLUNG VON PEPTIDEN ODER PROTEINEN

CRISTAUX DE GERMINATION POUR LA PREPARATION DE PEPTIDES OU DE PROTEINES

(84) Designated Contracting States:
**AT BE CH CY DE DK ES FI FR GB GR IE IT LI LU
MC NL PT SE**
Designated Extension States:
**LT RO SI**

(30) Priority: **30.06.1998 EP 98610020
15.07.1998 US 92882 P**

(43) Date of publication of application:
**18.04.2001 Bulletin 2001/16**

(73) Proprietor: **NOVO NORDISK A/S
2880 Bagsvaerd (DK)**

(72) Inventors:
• **MANIQUE, Flemming
DK-2750 Ballerup (DK)**
• **ILSOE, Christian
DK-3500 Vaerlose (DK)**

(74) Representative: **Plougmann & Vingtoft A/S
Sundkrogsgade 9,
P.O. Box 831
2100 Copenhagen Ø (DK)**

(56) References cited:
**EP-A- 0 096 631          EP-A- 0 582 351
WO-A-88/02633          WO-A-90/00176
GB-A- 766 994          GB-A- 766 995**

• **PATENT ABSTRACTS OF JAPAN vol. 017, no. 100
(C-1030), 26 February 1993 (1993-02-26) & JP 04
288013 A (KYORIN PHARMACEUT CO LTD), 13
October 1992 (1992-10-13)**
• **DEMATTEI R C ET AL: "CONTROLLING
NUCLEATION IN PROTEIN SOLUTIONS"
JOURNAL OF CRYSTAL GROWTH, vol. 122, no.
1 / 04, 2 August 1992 (1992-08-02), pages 21-30,
XP000306488 ISSN: 0022-0248**

**Description**

**FIELD OF THE INVENTION**

The present invention relates to a method for producing seeding microcrystals for the production of a peptide or protein, wherein the peptide or protein is selected from the group consisting of insulin and analogues or derivatives thereof, comprising providing an unseeded suspension of said peptide or protein and homogenising said suspension under pressure to result in peptide or protein microcrystals suitable for use as seeding microcrystals for the production of said peptide or protein..

**BACKGROUND OF THE INVENTION**

The "Lente" family of zinc insulin products are insulin zinc suspensions of the type originally developed in the 1950's with the aim of producing insulin preparations that would be able to cover diabetics' insulin requirement with a single daily injection ((see e.g. Jens Brange, *Galenics of Insulin,* 1987). Various Lente insulin products having different action profiles are available in the form of different combinations of amorphous and/or crystalline insulin particles from Novo Nordisk A/S, Denmark These include SEMILENTE, a suspension of amorphous insulin particles, ULTRALENTE, a suspension of crystalline insulin particles, and LENTE, which is a mixture of 30% amorphous and 70% crystalline insulin particles.

For several decades, seeding crystals for preparation of the "Lente" zinc insulin products have been prepared by the same basic freeze-drying method that was developed and patented in the early 1950's. This method, which is described in GB patent specification No. 766,994, involves the addition of freeze-dried amorphous insulin, typically beef insulin, to an insulin-containing crystallisation medium to result in the formation of a suspension of insulin microcrystals of a size of about 2-7 $\mu$m. This suspension, which is eventually used for the preparation of the final crystalline zinc insulin product, is filled into small vials (e.g. 10 ml), frozen in an alcohol/carbon dioxide mixture and stored frozen at a temperature at or below -18°C.

Although still in use, this method has a number of disadvantages:

1. It is based on the use of beef insulin, since it has until now not been possible to produce acceptable microcrystals of pure human insulin. As a result of the use of beef insulin nuclei for the formation of the microcrystals, the end product contains a small amount of beef insulin, which is undesirable.

2. The freeze-drying method requires a lyophiliser and subsequent shipping and storage at a temperature of no more than -18°C. This is expensive and requires a great deal of space.

3. The method of preparation is extremely difficult to perform in a sufficiently aseptic manner.

It would therefore be advantageous to be able to produce insulin seeding crystals using a method which does not suffer from the disadvantages of the known methods. It has now surprisingly been found that it is possible, using a relatively simple and inexpensive process, to produce insulin seeding crystals which are free of beef insulin, which can be stored at room temperature and which result in insulin preparations having advantageous properties in terms of e.g. crystal particle size and uniformity. Furthermore, it is also contemplated that this process will be applicable to the production of seeding crystals for other peptides and proteins, in particular peptides or proteins used as pharmaceuticals.

**SUMMARY OF THE INVENTION**

It is thus an object of the present invention to provide a novel method for the production of peptide or protein seeding crystals. More particularly, it is an object of the invention to provide a method for the production of insulin seeding crystals which does not require the use of beef insulin, which makes possible storage and transport without the need for expensive freeze-drying and storage at sub-zero temperatures, and which can be performed in a closed system so as to more readily allow the use of aseptic production methods.

Another object of the invention is to provide a method for the preparation of insulin seeding crystals for the production of crystalline zinc insulin suspensions having a narrow particle size distribution.

In its broadest aspect, the present invention thus relates to a method for producing seeding microcrystals for the production of a peptide or protein, wherein the peptide or protein is selected from the group consisting of insulin and analogues or derivatives thereof, comprising providing an unseeded suspension of said peptide or protein and homogenising said suspension under pressure to result in peptide or protein microcrystals suitable for use as seeding microcrystals for the production of said peptide or protein.

In a particular embodiment, the invention relates to a method for producing seeding microcrystals for the production of human insulin, said microcrystals being free of non-human pancreatic insulin, comprising providing an unseeded suspension of human insulin, said suspension being free of non-human pancreatic insulin, and homogenising said insulin suspension under pressure to result in human insulin microcrystals suitable for use as seeding microcrystals for the production of zinc insulin products.

Another aspect of the invention relates to a method for the production of a peptide or protein product wherein the peptide or protein is selected from the group consisting of insulin and analogues or derivatives thereof, said method comprising (i) providing a first unseeded suspension of said peptide or protein; (ii) homogenizing said unseeded suspension under pressure to result in microcrystals of said peptide or protein; and (iii) seeding a second unseeded suspension of said peptide or protein with microcrystals produced in step ii.

In a particular embodiment of this aspect of the invention, the peptide or protein product to be produced is a zinc insulin product, the first and second unseeded suspensions are suspensions of human insulin, and the seeding microcrystals are human insulin microcrystals.

## DETAILED DESCRIPTION OF THE INVENTION

As indicated above, the method of the invention is directed to the production of seedin microcrystals for peptides and proteins selected from the group consisting of insulin, and analogues and derivatives thereof. The peptide or protein is in particular human insulin or an analogue or derivative thereof as described below.

As used in the present text, the term "human insulin" is used to designate naturally occurring human insulin as well as insulin analogues and insulin derivatives. The term "insulin analogue" is used to designate a peptide with insulin activity, derived from a naturally occurring insulin by substitution of one or more amino acid residues, deletion of one or more amino acid residues and/or addition of one or more amino acid residues. An insulin or insulin analogue may optionally be in the form of an "insulin derivative", the term "derivative" referring to a peptide in which one or more of the amino acid residues of the parent peptide have been chemically modified, e.g. by alkylation, acylation, ester formation or amide formation. An "acylated insulin" (or insulin analogue) is an insulin (or insulin analogue) which has an acyl group in the $\varepsilon$-amino group of one or more amino acid residues, often a lysine residue.

As used herein, the term "non-human pancreatic insulin" refers to naturally occurring insulin from a non-human source, e.g. bovine or porcine insulin.

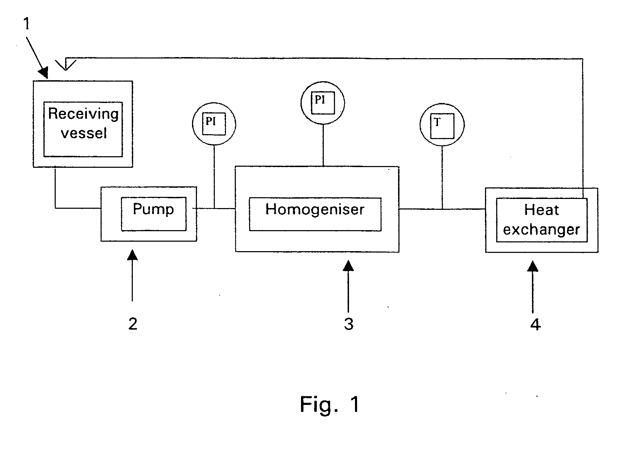

The basic principle of a presently preferred embodiment of the invention is shown schematically in Fig. 1. The apparatus of Fig. 1 comprises a receiving vessel 1, from which the insulin suspension is transferred by means of a pump 2 into a homogeniser 3. The homogeniser 3 comprises a valve with a very small opening through which the insulin suspension is pumped at a high pressure, e.g. about 1000 bars or higher. Upon exiting the valve, the insulin suspension is subjected to a sudden drop in pressure, which results in the rupture of the insulin crystals, i.e. a homogenisation effect. Since the insulin suspension is preferably subjected to multiple homogenisation cycles in order to result in a sufficiently homogenous suspension of microcrystals having the desired particle size and size distribution, and since the high pressure used in the homogeniser 3 results in an increase in the temperature of the suspension, the apparatus preferably also comprises a heat exchanger 4 downstream of the homogeniser 3 in order to reduce the suspension temperature. From the heat exchanger 4, the insulin suspension is returned to the receiving vessel 1 for further homogenisation cycles as necessary.

The temperature of the suspension increases according to the following equation:

$$\Delta T = P/(c \times \delta)$$

where:

$\Delta T$ = temperature increase (°C)
P = suspension pressure (N x m$^{-2}$)
$\delta$ = suspension density (g x m$^{-3}$)
c = specific heat (J x g$^{-1}$ x °C$^{-1}$)

The pressure and temperature are monitored during the process, and the above equation can be used in connection with design of the apparatus and regulation of the process.

In the method according to the invention, homogenisation is typically performed at a pressure of at least about 500 bars, preferably at least about 800 bars, more preferably at least about 1000 bars. In certain cases, the pressure may e.g. be at least about 1200 bars, for example up to about 1500 bars or more, even though such high pressures of above about 1000 bars are generally not believed to be necessary.

In a preferred embodiment, homogenisation of the suspension is performed using multiple homogenisation cycles, i.e. at least 2 cycles, since the use of multiple homogenisation cycles has been found to provide improved results, i.e. optimisation of seeding crystal size and uniformity. It is thus contemplated that it will normally be advantageous to use more than 2 cycles, such as 3, 4, 5, 6, 7, 8, 9 or 10 cycles or even more, e.g. in certain cases up to 15 or 20 cycles or perhaps even more than 20 cycles. The most advantageous number of homogenisation cycles will be determined by the person skilled in the art in each individual case based on factors such as the nature of the insulin suspension, the nature of the homogenisation apparatus used, the pressure used for homogenisation, and the desired insulin microcrystal particle size and size distribution.

Since, as indicated above, the high pressure used in the homogeniser results in an increase in the temperature of the suspension, the use of multiple homogenisation cycles is preferably accompanied by the use of a heat exchanger in order to reduce the suspension temperature, so that the suspension is maintained at a suitable temperature throughout the homogenisation process. Such heat exchangers are known in the art, and the person skilled in the art will readily be able to adapt the characteristics of the heat exchanger to suit the given process and apparatus. Preferably, the temperature of the recycled insulin suspension is maintained within the range of about 10-40°C, e.g. about 20-35°C.

Although the particle size of the resulting insulin microcrystals will vary depending on the intended use, suitable microcrystals will often have an average particle size, as defined by the longest diagonal of the crystals, in the range of about 0.5-4 $\mu$m, e.g. about 1-3 $\mu$m.

The result of the homogenisation process is human insulin microcrystals suitable for use as seeding microcrystals for the production of zinc insulin products, the microcrystals having the important feature of being free of non-human pancreatic insulin. For the production of zinc insulin products, the seeding microcrystals of the invention will be used in a conventional manner, i.e. an unseeded suspension of human insulin is seeded with the suspension of microcrystals produced as described above, and crystallisation is allowed to proceed in a manner known per se in the art. As is normal in the art, the precise amount of microcrystals to be added to a given unseeded insulin suspension may be determined empirically.

The invention will be further illustrated by the following non-limiting examples.

EXAMPLES

Materials and methods

Using the basic homogenisation process and apparatus described above, i.e. a recirculating homogeniser equipped with a heat exchanger, a number of experiments were performed to test the effect of the number of homogenisation cycles as well as homogenisation pressure and time.

The apparatus used was a Rannie high pressure homogeniser, model LAB 10,51 VH (series 1.89239), equipped with a ceramic valve, type SEO 719685. The capacity of the homogeniser was 80 l/h at a pressure of 1000 bars. A centrifugal pump provided an inlet pressure of 4.5-5 bars. The heat exchanger for these experiments used a cooling water temperature of about 20°C. However, since the capacity of the heat exchanger was insufficient in relation to this particular homogeniser, the outlet temperature of the insulin suspension was somewhat higher at the maximum homogeniser output, i.e. about 28-29°C, but slightly lower at a lower homogeniser output of about 65 l/h, i.e. about 24-28°C. The receiving vessel comprised a 100 l pressure tank and a small conic vessel with a volume of about 3 l.

The insulin suspension used for producing the microcrystals was a pooled batch (2 x 20 l) of unseeded ULTRALENTE HM(ge), 100 U/ml, from Novo Nordisk A/S.

EXAMPLE 1

A 10 l portion of the pooled batch of the ULTRALENTE insulin suspension was homogenised at a pressure of 1000 bars, the suspension being recirculated for multiple homogenisation cycles as described above, resulting in a gradually increased degree of homogenisation. A flow rate of 80 l/h was used. A total of 18 homogenisation cycles were performed, and samples were taken for the first 10 cycles and after the final cycle. The temperature of the insulin suspension was measured in the outlet conduit between the homogeniser and the heat exchanger. The times and measured temperatures for the various cycles were as indicated in Table 1 below:

Table 1

| Example number | Number of homogenisation cycles | Time from start (minutes) | Temperature (°C) |
|---|---|---|---|
| 1-0 | 0 | 0 | 12.5 |

(continued)

| Example number | Number of homogenisation cycles | Time from start (minutes) | Temperature (°C) |
|---|---|---|---|
| 1-1 | 1 | 5 | 29 |
| 1-2 | 2 | 12 | 29 |
| 1-3 | 3 | 19 | 28.3 |
| 1-4 | 4 | 26 | 29.5 |
| 1-5 | 5 | 33 | 29.5 |
| 1-6 | 6 | 40 | 29.8 |
| 1-7 | 7 | 47 | 29.5 |
| 1-8 | 8 | 54 | 29.2 |
| 1-9 | 9 | 61 | 28.1 |
| 1-18 | 18 | 122 | 28.1 |

A number of the samples were investigated by microsope, and the following observations were made:

Example 1-0: mostly whole and sharp-edged rhombohedric crystals having a size of about 3-80 $\mu$m; some broken crystals and crystal fragments.

Example 1-1: still many whole rhombohedric crystals having a size of about 20-40 $\mu$m, but also many small crystal fragments with a size of 3 $\mu$m or less.

Example 1-2: still some whole rhombohedric crystals with a size of up to about 20 $\mu$m as well as a few larger crystal agglomerations of up to about 40 $\mu$m; even more small crystal fragments of 3 $\mu$m or less.

Example 1-18: small microcrystals of about 1 $\mu$m or less; a few crystal fragments of up to about 10 $\mu$m; no whole rhombohedric crystals.

EXAMPLE 2

A 5 l portion of the pooled batch of the ULTRALENTE insulin suspension was homogenised at a pressure of 1000 bars, the suspension being recirculated for multiple homogenisation cycles as described above, using a flow rate of 65 I/h. A total of 10 homogenisation cycles were performed, and samples were taken after each cycle. In this case, instead of being led directly back to the receiving vessel from the heat exchanger, the suspension was collected after each cycle. A sample was taken from each portion, and the remainder of the portion was returned to the receiving vessel for the next homogenisation cycle. The times and measured temperatures were as follows:

Table 2

| Example number | Number of homogenisation cycles | Time from start (minutes) | Temperature (°C) |
|---|---|---|---|
| 2-0 | 0 | 0 | - |
| 2-1 | 1 | - | 24.5 |
| 2-2 | 2 | - | 26.3 |
| 2-3 | 3 | - | 27.1 |
| 2-4 | 4 | 15 | 27.7 |
| 2-5 | 5 | - | 27.8 |
| 2-6 | 6 | - | 28-0 |
| 2-7 | 7 | - | 28.0 |
| 2-8 | 8 | - | 28.0 |

(continued)

| Example number | Number of homogenisation cycles | Time from start (minutes) | Temperature (°C) |
|---|---|---|---|
| 2-9 | 9 | - | - |
| 2-10 | 10 | 30 | - |

EXAMPLE 3

In order to investigate the effect of the homogenisation pressure, tests were performed at 1400-1500 bars, with a total of 9 homogenisation cycles. Due to the increased pressure and the accompanying increased temperature of the suspension, the flow rate was further reduced to 54 1/h to allow the heat exchanger to provide a sufficiently reduced temperature The batch size in this case was 3 1. The temperature of the suspension was maintained at about 26-29°C.

Table 3

| Example number | Number of homogenisation cycles | Time from start (minutes) | Temperature (°C) |
|---|---|---|---|
| 3-0 | 0 | 0 | 16.3 |
| 3-9 | 9 | 30 | 28.6 |

EXAMPLE 4

The same procedure as in Example 3 was used, with the exception that the ULTRALENTE insulin suspension in this case had formed divergent crystals ("roses") during crystallisation. The batch size was 2 1, and the homogenisation time was therefore reduced correspondingly to a total of 21 minutes.

Table 4

| Example number | Number of homogenisation cycles | Time from start (minutes) | Temperature (°C) |
|---|---|---|---|
| 4-0 | 0 | 0 | - |
| 4-9 | 9 | 21 | 27.7 |

EXAMPLE 5

*Seeding experiments with selected batches of microcrystals*

Seeding experiments were performed to test selected batches of the human insulin microcrystals prepared as described above. As a reference, a standard bovine microcrystal seeding batch was also tested. These experiments were performed using 1 l batches of ULTRALENTE (40 U/ml). Crystallisation was performed using propeller agitation for a period of 20 hours. The results are shown in Table 5 below.

Table 5

| Microcrystals of Example number | Average crystal size ($\mu$m) | 10%-90% Deviation ($\mu$m) |
|---|---|---|
| Reference | 28 | 16 |
| 1-18 | 23 | 15 |
| 2-10 | 26 | 17 |
| 3-9 | 26 | 17 |
| 4-9 | 27 | 16 |

It may be seen from the results in Table 5 that the human insulin microcrystals prepared according to the invention gave an insulin crystal size and deviation comparable to that obtained using the standard bovine microcrystals, and that the five seeding batches gave largely identical results.

The five insulin batches prepared as described above were in addition analysed with regard to a number of other parameters, including pH, insulin strength, A+M+B component, percentage of amorphous insulin, content of methyl para-hydroxybenzoate, dimer and polymer content, acidic and neutral desamidoins content, and zinc content. It was found that insulin batches prepared using microcrystals according to the invention were generally comparable to insulin prepared using the standard bovine microcrystals.

Since it is known that the crystallisation time and type of agitation can have an effect on the appearance of the rhombohedrons that are formed, a single batch prepared according to the invention (Example 3-9) was used for seeding tests in which the crystallisation time and type of agitation were varied. With regard to agitation, no substantial differences were observed between crystals obtained using propeller agitation and agitation using "cradle movements". With regard to crystallisation time, it was found that 4 hours was sufficient, i.e. a crystallisation time of 20 hours was found to be unnecessary. There was a tendency for the best results to be obtained using propeller agitation and a crystallisation time of 4 hours, as this led the least amount of deviating crystals.

*Conclusion*

It may be concluded that pure microcrystals of human insulin can be produced by high pressure homogenisation of an unseeded ULTRALENTE HM(ge) preparation. This method results in microcrystals in the form of small crystal fragments with a particle size of about 1-2 $\mu$m and some larger fragments with a particle size of up to about 10 $\mu$m.

Varying the pressure from 1000 bars to about 1500 bars did not have any noticeable effect on the microcrystals. On the other hand, the number of homogenisation cycles has an effect, at least up to a point, an increased number of cycles resulting in a more uniform microcrystal suspension with a larger proportion of microcrystals having a size of about 1-2 $\mu$m and a smaller proportion of larger crystal fragments and whole rhombohedric crystals.

However, the number of homogenisation cycles required to result in a given degree of homogenisation is also related to the homogenisation time per cycle.

The increase in temperature of the suspension measured in these experiments as a result of the homogenisation process did not appear to effect the microcrystals in terms of chemical degradation. Temperature regulation can be optimised by suitable changes in e.g. the design and capacity of the heat exchanger.

The variation in the particle size of the microcrystals produced in these tests, and thus the variation in particle size of the zinc insulin product prepared using the microcrystals, could, if desired or necessary, be reduced by means of e.g. sedimentation or centrifugation.

The seeding qualities of the microcrystals produced according to the invention have been shown to be acceptable, since the microcrystals result in zinc insulin products with rhombohedric crystals having an acceptable crystal size.

## Claims

1. A method for producing seeding microcrystals for the production of a peptide or protein, wherein the peptide or protein is selected from the group consisting of insulin and analogues or derivatives thereof, comprising providing an unseeded suspension of said peptide or protein and homogenising said suspension under pressure to result in peptide or protein microcrystals suitable for use as seeding microcrystals for the production of said peptide or protein.

2. The method of claim 1 for producing seeding microcrystals for the production of human insulin, said microcrystals being free of non-human pancreatic insulin, comprising providing an unseeded suspension of human insulin, said suspension being free of non-human pancreatic insulin, and homogenising said insulin suspension under pressure to result in human insulin microcrystals suitable for use as seeding microcrystals for the production of zinc insulin products.

3. The method of claim 1 or 2, wherein homogenisation is performed at a pressure of at least 500 bars.

4. The method of any of claims 1-3, wherein homogenisation is performed using multiple homogenisation cycles.

5. The method of claim 4, wherein the temperature of the suspension during said homogenizing step is controlled using a heat exchanger.

6. The method of claim 5, wherein the temperature of the suspension during said homogenizing step is maintained within the range of 10-40°C.

7. A method for producing microcrystals having an average particle size, as defined by the longest diagonal of the crystals, in the range of about 0.5-4 $\mu$m by applying the steps as described in any of claims 1-6.

**8.** A method for the production of a peptide or protein product wherein the peptide or protein is selected from the group consisting of insulin and analogues or derivatives thereof, said method comprising (i) providing a first unseeded suspension of said peptide or protein; (ii) homogenizing said unseeded suspension under pressure to result in microcrystals of said peptide or protein; and (iii) seeding a second unseeded suspension of said peptide or protein with microcrystals produced in step ii.

**9.** The method of claim 8, wherein the peptide or protein product to be produced is a zinc insulin product, the first and second unseeded suspensions are suspensions of human insulin, and the seeding microcrystals are human insulin microcrystals.

**Patentansprüche**

**1.** Verfahren zur Herstellung von Keimmikrokristallen zur Herstellung eines Peptids oder Proteins, worin das Peptid oder Protein aus der Gruppe bestehend aus Insulin und Analogen oder Derivaten davon ausgewählt ist, welches Verfahren die Bereitstellung einer mit Keimen nicht-versehenden Suspension des Peptids oder Proteins und die Homogenisierung der Suspension unter Druck umfasst, um Peptid- oder Protein-Mikrokristalle zu erzielen, die zur Anwendung als Keimmikrokristalle zur Herstellung des Peptids oder Proteins geeignet sind.

**2.** Verfahren nach Anspruch 1 zur Herstellung mit Keimen versehender Mikrokristalle zur Herstellung von Human-Insulin, indem die Mikrokristalle frei von nicht-humanem Bauchspeicheldrüsen-Insulin sind, umfassend die Bereitstellung einer mit Keimen nicht-versehenden Suspension von Human-Insulin, wobei die Suspension frei von nicht-humanem Bauchspeicheldrüsen-Insulin ist, und die Homogenisierung der Insulinsuspension unter Druck, um Human-Insulin-Mikrokristalle zu erzielen, die für die Anwendung als mit Keimen versehenden Mikrokristalle zur Herstellung von Zink-Insulinprodukten geeignet sind.

**3.** Verfahren nach Anspruch 1 oder 2, worin die Homogenisierung bei einem Druck von mindestens 500 bar ausgeführt wird.

**4.** Verfahren nach irgendeinem der Ansprüche 1-3, worin die Homogenisierung unter Anwendung mehrerer Homogenisierungszyklen ausgeführt wird.

**5.** Verfahren nach Anspruch 4, worin die Temperatur der Suspension während der Homogenisierungsstufe unter Anwendung eines Wärmeaustauschers geregelt wird.

**6.** Verfahren nach Anspruch 5, worin die Temperatur der Suspension während der Homogenisierungsstufe innerhalb des Intervalls von 10-40°C aufrechterhalten wird.

**7.** Verfahren zur Herstellung von Mikrokristallen mit einer durchschnittlichen Partikelgröße, wie durch die längste Diagonale der Kristalle definiert, innerhalb des Intervalls von ungefähr 0,5-4 μm unter Anwendung der Stufen, wie in irgendeinem der Ansprüche 1-6 beschrieben.

**8.** Verfahren zur Herstellung eines Peptid- oder Proteinprodukts, worin das Peptid oder Protein aus der Gruppe bestehend aus Insulin und Analogen oder Derivaten davon ausgewählt ist, wobei das Verfahren (i) die Bereitstellung einer ersten mit Keimen nicht-versehenden Suspension des Peptids oder Proteins; (ii) die Homogenisierung der mit Keimen nicht-versehenden Suspension unter Druck, um Mikrokristalle des Peptids oder Proteins zu erzielen; und (iii) das Besamen einer zweiten mit Keimen nicht-versehenden Suspension des Peptids oder Proteins mit Mikrokristallen, die unter Stufe ii hergestellt werden, umfasst.

**9.** Verfahren nach Anspruch 8, worin das herzustellende Peptid- oder Proteinprodukt ein Zink-Insulinprodukt ist, die ersten und zweiten mit Keimen nicht-versehenden Suspensionen Suspensionen aus Human-Insulin sind, und die mit Keimen versehenden Mikrokristalle Human-Insulin-Mikrokristalle sind.

**Revendications**

**1.** Méthode pour produire des microcristaux d'ensemencement pour la production d'un peptide ou d'une protéine, où le peptide ou la protéine est choisi(e) dans le groupe consistué par l'insuline et les analogues ou dérivés de celle-ci, comprenant l'approvisionnement en une suspension non ensemencée dudit peptide ou de ladite protéine et l'homogénéisation de ladite suspension sous pression pour donner des microcristaux peptidiques ou protéiques appropriés pour usage en tant que microcristaux d'ensemencement pour la production dudit peptide ou de ladite protéine.

2. La méthode de la revendication 1 pour la production de microcristaux d'ensemencement pour la production d'insuline humaine, lesdits microcristaux étant dépourvus d'insuline pancréatique non humaine, comprenant une suspension non ensemencée d'insuline humaine, ladite suspension étant dépourvue d'insuline pancréatique non humaine, et l'homogénéisation de ladite suspension d'insuline sous pression pour donner des microcristaux d'insuline humaine appropriés pour usage en tant que microcristaux d'ensemencement pour la production de produits d'insuline zinc.

3. La méthode selon la revendication 1 ou 2, où l'homogénéisation est effectuée à une pression d'au moins 500 bars.

4. La méthode selon une quelconque des revendications 1-3, où l'homogénéisation est effectuée en utilisant des cycles multiples d'homogénéisation.

5. La méthode de la revendication 4, où la température de la suspension durant ladite étape d'homogénéisation est contrôlée en utilisant un échangeur calorique.

6. La méthode de la revendication 5, où la température de la suspension durant ladite étape d'homogénéisation est maintenue dans la gamme de 10-40°C.

7. Méthode pour produire des microcristaux présent une taille moyenne de particule, telle que définie par la plus longue diagonale des cristaux, dans la gamme de 0,5-4 $\mu$m, en mettant en application les étapes telles que décrites dans une quelconque des revendications 1-6.

8. Méthode pour la production d'un produit peptidique ou protéique, où le peptide ou la protéine est choisi(e) dans le groupe consitué par l'insuline et les analogues ou dérivés de celle-ci, ladite méthode comprenant (i) l'approvisionnement en une première suspension non ensemencée dudit peptide ou de ladite protéine ; (ii) l'homogénéisation de ladite suspension non ensemencée sous pression pour donner des microcristaux dudit peptide ou de ladite protéine ; et (iii) l'ensemencement d'une deuxième suspension non ensemencée dudit peptide ou de ladite protéine avec les microcristaux produits dans l'étape ii.

9. La méthode selon la revendication 8, où le produit peptidique ou protéique devant être produit est un produit d'insuline zinc, la première et la deuxième suspensions non ensemencées sont des suspensions d'insuline humaine, et les microcristaux sont des microcristaux d'insuline humaine.

Fig. 1